# EUROPÄISCHE PATENTSCHRIFT

(11) **EP 1 150 657 B1**
(45) Veröffentlichungstag und Bekanntmachung des Hinweises auf die Patenterteilung: **10.09.2003**
(21) Anmeldenummer: 00907522.7
(22) Anmeldetag: 09.02.2000
(51) Int. Cl.: A61K 9/00, A61P 25/02, A61P 25/16, A61P 25/18, A61P 25/24, A61P 25/28

(54) **SPRAY, ENTHALTEND UBICHINON Qn**
SPRAY CONTAINING UBIQUINONE Qn
SPRAY CONTENANT DU COENZYME Qn.

(30) Priorität: 11.02.1999 DE 19905880
(43) Veröffentlichungstag der Anmeldung: 07.11.2001
(73) Patentinhaber: MSE Pharmazeutika GmbH, 61348 Bad Homburg (DE)
(72) Erfinder: ENZMANN, Franz, D-61348 Bad Homburg v.d.H. (DE)
(74) Vertreter: Werner, Hans-Karsten, Dr.Dipl.-Chem.
(86) Internationale Anmeldenummer: EP0001012
(87) Internationale Veröffentlichungsnummer: WO00047185

(56) Entgegenhaltungen:
- WO-A-96/17626
- WO-A-97/42938
- FR-A- 2 703 591
- US-A- 5 660 835
- DATABASE WPI Week 7750 Derwent Publications Ltd., London, GB; AN 1977-89050y XP002138662 "Coenzyme-Q used to treat headaches, dizziness etc.. administered orally or parenterally" & JP 52 130922 A (EISA CO LTD), 2. November 1977 (1977-11-02)
- DATABASE MEDLINE [Online] US NATIONAL LIBRARY OF MEDICINE (NLM), BETHESDA, MD, US YAMAZAKI M ET AL: "A case of mitochondrial encephalomyopathy with schizophrenic psychosis, dementia and neuroleptic malignant syndrome." retrieved from STN Database accession no. 92257815 XP002138736 & RINSHO SHINKEIGAKU. CLINICAL NEUROLOGY, (1991 NOV) 31 (11) 1219-23. ,
- WOOD W, SHEPERD J, CHONG B, MEINWALD J: "Ubiquinone-O in defensive spray of African millipede" NATURE, Bd. 253, 1975, Seiten 625-626, XP002138410

## Beschreibung

Ubichinone sind prenylierte Chinone und in der Tier- und Pflanzenwelt weit verbreitet. Es handelt sich um Derivate von 2,3-Dimethoxy-5-methyl-1,4-benzochinon, die in Sechsstellung linear verknüpfte Isopreneinheiten aufweisen. Je nach Anzahl der Isopreneinheiten werden die Ubichinone als Q-1, Q-2, Q-3 usw. bezeichnet. Bei den meisten Säugetieren einschließlich des Menschen überwiegt Q-10 (2,3-Dimethoxy-5-methyl-6-decaprenyl-1,4-benzochinon). Ubichinone dienen als Elektronenüberträger in der Atmungskette, sie sind beteiligt an der zyklischen Oxidation und Reduktion von Substraten im Zitronensäurezyklus. Ubichinone Qₙ sind Voraussetzung für die Energieversorgung aller Zellen. Der oxidative Stress, der unter anderem durch hohen Sauerstoffverbrauch entsteht, löst Schäden an den Membranen von Mitchondrien und Zellen aus, die zu akuten oder degenerativen Störungen des Nervensystems führen. Das Nervensystem hat einen sehr hohen Energiebedarf für die Signalübertragung durch Membranpotentialaufbau, Ionenkanalsteuerung sowie durch Neuropeptid- und Neurotransmitter-Vesikelbildung.

Ubichinon Q-10 (auch Coenzym Q-10 genannt) wurde bisher in der Therapie von Herzerkrankungen verwendet.

Die Patentdokumente WO 97/42938 und US-A-5 660 835 offenbaren beide ein Spray enthaltend Ubidecarerion.

Gegenstand der Erfindung ist ein Spray enthaltend Ubichinon Qₙ oder Ubichinon Qₙ-Vorstufen im amorphen Zustand als wässrige kolloidale Dispersion zusammen mit üblichen Hilfsstoffen.

Mit Ubichinon Qₙ-Vorstufen werden Verbindungen bezeichnet, die im Körper zu Ubichinon Qₙ umgewandelt werden. Dies sind zum einen die Ubihydrochinone, die mit den Ubichinonen im Gleichgewicht stehen sowie einfache Ester der Ubihydrochinone mit kurzkettigen Carbonsäuren mit 1-10 C-Atomen, beispielsweise Essigsäure-, Propionsäure- oder Buttersäureester. Diese Vorstufen werden nach der Applikation in die entsprechenden Ubichinone umgewandelt.

Bevorzugt wird Ubichinon Q-10 verwendet, da dies das Haupt-Ubichinon beim Menschen ist.

Bevorzugt handelt es sich bei dem erfindungsgemäßen Spray um ein Mund- oder Nasenspray, so dass die Applikation des Ubichinon Qₙ oder Ubichinon Qₙ-Vorstufen inhalativ oder intranasal erfolgen kann. Das erfindungsgemäße Spray eignet sich insbesondere zur Behandlung von Schmerzzuständen wie bei Migräne und Neuropathie, bei neuralen Störungen wie Depressionen, Psychosen, Konzentrationsstörungen und bei neurodegenerativen Erkrankungen wie Alzheimer, Parkinson, Huntington, Multipier Sklerose und Cerebraler Parese. Die Anwendung als Mund- oder Nasenspray scheint dabei den Transport des Ubichinon Qₙ bzw. seiner Vorstufen durch die Bluthirnschranke zu beschleunigen. Es wurde gefunden, daß bei oraler Verabreichung von 600 bis 800 mg Q-10 in Form von Kapseln praktisch keine Wirkung bei Migräne zeigte, während eine Nasalzubereitung bereits bei kleiner Menge effektiv Schmerzen bei Migräne und Spannungskopfschmerzen lindern konnte. So genügten Dosen von etwa 20 mg für eine signifikante Schmerzlinderung. Prinzipiell können jedoch Dosen bis zu 1000 mg eingesetzt werden.

Ubichinone sind lipophile Substanzen, die in Wasser praktisch nicht löslich sind. Besonders hohe Effektivität wurde jedoch gefunden, wenn das Ubichinon Qn oder seine Vorstufen in einer wässrigen Dispersion vorliegen. Wässrige kolloidale Dispersionen sind besonders bevorzugt. Die Herstellung entsprechender Dispersionen ist in der WO 95/05164 sowie in der damit verbundenen DE-A-43 27 063 beschrieben.

## Patentansprüche

1. Spray enthaltend amorphes Ubichinon Q-10 als wässrige kolloidale Dispersion zusammen mit üblichen Hilfsstoffen.

2. Spray nach Anspruch 1, **dadurch gekennzeichnet, dass** es sich um ein Mund- oder Nasenspray handelt.

3. Verwendung des Sprays nach einem der Ansprüche 1 oder 2, zur Herstellung eines Medikaments zur Behandlung von Schmerzzuständen durch neurale Störungen, insbesondere bei Migräne und Neuropathie.

4. Verwendung des Sprays nach einem der Ansprüche 1 oder 2, zur Herstellung eines Medikaments zur Behandlung von neuralen Störungen wie Depressionen, Psychosen, Konzentrationsstörungen.

5. Verwendung des Sprays nach einem der Ansprüche 1 oder 2, zur Herstellung eines Medikaments zur Behandlung von neurodegenerativen Erkrankungen wie Alzheimer, Parkinson, Huntington, Multiple Sklerose und Cerebrale Parese.

## Claims

1. A spray comprising amorphous ubiquinone Q-10 as an aqueous colloidal dispersion together with usual auxiliaries.

2. The spray according to claim 1, **characterized by** being an oral or nasal spray.

3. Use of the spray according to either of claims 1 or 2 for preparing a medicament for the treatment of pain conditions from neural disorders, especially in migraine and neuropathy.

4. Use of the spray according to either of claims 1 or 2 for preparing a medicament for the treatment of neural disorders such as depressions, psychoses, lack of concentration.

5. Use of the spray according to either of claims 1 or 2 for preparing a medicament for the treatment of neurodegenerative diseases, such as Alzheimer's, Parkinson's, Huntington's, multiple sclerosis and cerebral paresis.

## Revendications

1. Spray contenant de l'ubiquinone Q₁₀ amorphe sous forme de dispersion aqueuse colloïdale mélangée à des adjuvants usuels.

2. Spray selon la revendication 1, **caractérisé en ce qu'**il s'agit d'un spray buccal ou nasal.

3. Utilisation du spray selon une des revendications 1 ou 2, pour préparer un médicament destiné au traitement des états douloureux dus à des troubles du système nerveux central, notamment en cas de migraine ou de neuropathie.

4. Utilisation du spray selon une des revendications 1 ou 2, pour préparer un médicament destiné au traitement des maladies du système nerveux central telles que les dépressions, les psychoses, les troubles de la concentration.

5. Utilisation du spray selon une des revendications 1 ou 2, pour préparer un médicament destiné au traitement des maladies neurodégénérescentes telles que les maladies d'Alzheimer, de Parkinson, d'Huntington, la sclérose multiple et la parésie cérébrale.
